# FASCICULE DE BREVET EUROPEEN

(11) **EP 0 729 750 B1**
(45) Date de publication et mention de la délivrance du brevet: **24.03.2004**
(21) Numéro de dépôt: 95402677.9
(22) Date de dépôt: 28.11.1995
(51) Int. Cl.: A61K 31/00, A61K 31/41, A61K 31/495, A61K 31/44, A61K 7/48

(54) **Composition cosmétique, pharmaceutique ou dermatologique contenant un antagoniste TNF-alpha.**
Kosmetische, pharmazeutische oder dermatologische Zusammensetzung, sowie die Zusammensetzung Alpha-TNFAntagonisten enthaltend
Alpha-TNF antagonists in a cosmetic, pharmaceutical or dermatological composition.

(30) Priorité: 28.12.1994 FR 9415796
(43) Date de publication de la demande: 04.09.1996
(73) Titulaire: L'OREAL, 75008 Paris (FR)
(72) Inventeur: de Lacharriere, Olivier, F-75015 Paris (FR); Breton, Lionel, F-78000 Versailles (FR); Cohen, Catherine, F-75013 Paris (FR)
(74) Mandataire: Lhoste, Catherine

(56) Documents cités:
- EP-A- 0 299 457
- EP-A- 0 413 528
- EP-A- 0 428 754
- EP-A- 0 524 108
- EP-A- 0 659 416
- WO-A-93/07902
- WO-A-93/10755
- DE-A- 4 033 563
- FR-A- 2 596 986
- FR-A- 2 685 202
- GB-A- 2 006 771
- NL-A- 256 310
- US-A- 3 435 114
- US-A- 4 808 612
- US-A- 4 902 685
- US-A- 4 902 800
- US-A- 5 281 608
- DATABASE WPI Week 8927 Derwent Publications Ltd., London, GB; AN 89-197157 XP002004785 & JP-A-01 135 726 (NONOGAWA SHOJI YG)
- PATENT ABSTRACTS OF JAPAN vol. 13, no. 569 (C-666) & JP-A-01 238509 (SHISEIDO COMPANY LIMITED), 22 Septembre 1989,
- PATENT ABSTRACTS OF JAPAN vol. 16, no. 315 (C-0961) & JP-A-04 089428 (TSUJITA SATOSHI), 23 Mars 1992,
- PATENT ABSTRACTS OF JAPAN vol. 8, no. 212 (C-244) & JP-A-59 098014 (TAISHO SEIYAKU KK), 6 Juin 1984,
- CHEMICAL ABSTRACTS, vol. 121, no. 25, 19 Décembre 1994 Columbus, Ohio, US; abstract no. 298969, CHABOT-FLETCHER ET AL.: "interleukin-8 production is regulated by protein kinase c in human keratinocytes" XP002004783 & J. INVEST. DERMATOL., vol. 103, no. 4, 1994, pages 509-515,
- CHEMICAL ABSTRACTS, vol. 103, no. 19, 11 Novembre 1985 Columbus, Ohio, US; abstract no. 153604, GRISWOLD ET AL.: "modulation of macrophage-lymphocyte interactions by the antiarthritic gold compound, auranofin." XP002004784 & J. RHEUMATOL., vol. 12, no. 3, 1985, pages 490-497,

## Description

La présente invention se rapporte à l'utilisation d'un antagoniste d'histamine, d'un antagoniste d'interleukine 1 et/ou d'un antagoniste de TNF-alpha dans une composition cosmétique, pharmaceutique ou dermatologique à application topique, destinée notamment au traitement des peaux sensibles, ainsi qu'à une composition contenant un antagoniste de TNF-alpha en vue de diminuer voire éliminer les effets irritants de certains produits et notamment de certains actifs utilisés dans le domaine cosmétique, pharmaceutique ou dermatologique.

Il est connu que certaines peaux sont plus sensibles que d'autres. Les symptômes des peaux sensibles étaient jusqu'à présent mal caractérisés et le problème de ces peaux était, de ce fait, mal défini personne ne connaissait exactement le processus mis en cause dans la sensibilité -hyperréactivité cutanée non allergique- de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques.

Certains tests ont été essayés pour tenter de cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217. Mais ces tests ne permettaient pas de caractériser complètement les peaux sensibles.

Par ailleurs, on assimilait les peaux sensibles à des peaux allergiques.

Du fait que l'on connaissait mal les caractéristiques des peaux sensibles, il était jusqu'à présent très difficile de les traiter, et on les traitait indirectement, par exemple en limitant dans les compositions cosmétiques ou dermatologiques l'emploi de produits à caractère irritant tels que les tensioactifs, les conservateurs ou les parfums ainsi que certains actifs.

La demanderesse a réalisé de nombreux tests cliniques et a su déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique.

La demanderesse a maintenant trouvé que les peaux sensibles pouvaient être scindées en deux grandes formes cliniques, les peaux irritables et les peaux intolérantes.

Une peau irritable est une peau qui réagit par un prurit, c'est-à-dire par des démangeaisons ou par des picotements à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres, ou à une peau qui présente un érythème.

Une peau intolérante est une peau qui réagit par des sensations d'échauffernent, de tiraillements, par un prurit c'est-à-dire par des démangeaisons ou des picotements, par des fourmillements et/ou des rougeurs, à différents facteurs tels que l'environnement, les émotions, les aliments. En général, ces signes sont associés à un érythème et à une peau avec ou sans dartres.

Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions eUou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à l'eau dure à forte concentration en calcaire et/ou aux variations de température.

Pour déterminer si une peau est sensible ou non, la demanderesse a également mis au point un test. En effet, après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm² de peau 0,05 ml d'une crème contenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

Jusqu'à présent, la capsaïcine était utilisée comme médicament, en particulier pour traiter les douleurs du zona. La capsaïcine provoque un relargage des neuropeptides, et en particulier des tachykinines qui proviennent de terminaisons nerveuses de l'épiderme et du derme. La demanderesse a constaté que le schéma physiopathologique commun à tous les états des peaux sensibles était lié à une grande aptitude à libérer des tachykinines et plus particulièrement de la substance P dans la peau. On sait, en outre, que la substance P libérée par les terminaisons sensitives épidermiques induit une cascade d'événements biochimiques dont les premières étapes concernent les mastocytes. La fixation de la substance P sur les récepteurs mastocytaires induit une libération de nombreux médiateurs pro-inflammatoires parmi lesquels l'histamine, la sérotonine, l'interleukine 1 (IL1), l'interleukine 6 (IL6), l'interleukine 8 (IL8) et le Tumor Necrosis Factor-alpha (TNFalpha).

Par ailleurs, la demanderesse a trouvé que les peaux sensibles telles que définies ci-dessus sont en outre caractérisées par des taux élevés d'Interleukine 1 et/ou d'histamine dans les couches superficielles de l'épiderme. Ces taux sont d'autant plus élevés que l'état de réactivité de la peau est important.

La demanderesse a maintenant découvert que les caractéristiques essentielles des peaux sensibles (réactions d'irritation et d'intolérance cutanée) étaient liées à la libération de substance P et par voie de conséquence à la libération d'histamine, d'interleukine 1 et en particulier de TNF-alpha et que les antagonistes d'interleukine 1 et/ou les antagonistes de TNF-alpha et/ou les antagonistes d'histamine pouvaient être utilisés dans le traitement préventif et/ou curatif des peaux sensibles.

On entend par « antagonistes » de TNF-alpha toutes substances susceptibles d'inhiber la libération et/ou la synthèse et/ou la fixation réceptorielle respectivement de TNF-alpha.

En outre, la demanderesse a constaté que l'adjonction d'antagonistes de TNF-alpha dans des compositions cosmétiques, pharmaceutiques ou dermatologiques, à application topique, contenant des produits irritants (alpha-hydroxy-acides, rétinoïdes, peroxyde de benzoyle,...) permettait également de diminuer, voire de supprimer les réactions d'irritations habituellement provoquées par ces produits. Ces réactions d'irritations se traduisent dans les instants qui suivent l'application par des sensations dysesthésiques (échauffement, sensations de brûlures, démangeaisons ou prurit, sensations de picotements, de tiraillements...), et/ou par des rougeurs, et/ou par de l'oedème. Ces états d'irritation peuvent également se traduire à distance de l'application par la persistance, l'apparition ou la réapparition des sensations dysesthésiques sus-citées et/ou par des rougeurs et/ou des squames ; ces états d'irritation de la peau peuvent prendre l'aspect de plaques de xérose cutanée et/ou de dartres.

Par ailleurs, la libération d'histamine induite par l'inflammation neurogène provoque une vasodilatation qui se traduit par un érythème, un oedéme et un prurit. Aussi, l'adjonction d'antagonistes d'histamine spécifiques des récepteurs H1, dans des compositions cosmétiques, pharmaceutiques ou dermatologiques irritantes permet également de diminuer, voire de supprimer les réactions d'irritations habituellement provoquées par ces produits.

Pour traiter les peaux sensibles, la demanderesse a donc envisagé d'utiliser des antagonistes de TNF-alpha. Elle a en effet constaté de manière surprenante que l'incorporation d'antagonistes de TNF-alpha dans une composition cosmétique, pharmaceutique ou dermatologique permet d'éviter l'irritation et/ou les sensations dysesthésiques et/ou les prurits de la peau et/ou des muqueuses.

La présente invention a donc pour objet l'utilisation d'au moins un composé choisi parmi les antagonistes de TNF-alpha et leurs associations, dans une composition contenant un milieu cosmétiquement, dermatologiquement ou pharmaceutiquement acceptable pour traiter les peaux sensibles.

La présente invention a encore pour objet l'utilisation dans une composition topique d'au moins un composé choisi parmi les antagonistes de TNF-alpha et leurs associations pour prévenir et/ou lutter contre les irritations cutanées et/ou les dartres et/ou les érythèmes et/ou les sensations dysesthésiques et/ou les sensations d'échauffement et/ou les prurits et/ou les picotements et/ou les fourmillements et/ou les inconforts et/ou les tiraillements de la peau et/ou des muqueuses.

Un milieu cosmétiquement, dermatologiquement ou pharmaceutiquement acceptable est un milieu qui est compatible avec la peau, le cuir chevelu, les ongles, les muqueuses. La composition contenant un antagoniste de TNF-alpha peut donc être appliquée sur le visage, le cou, les cheveux et les ongles, ou toute autre zone cutanée du corps comme les grands plis (régions axillaires, sous-mammaires, plis du coude etc.).

Pour qu'une substance soit reconnue comme un antagoniste réceptoriel d'histamine, d'interleukine 1 (IL1) ou de TNF-alpha, elle doit répondre notamment à la caractéristique suivante :
- avoir une activité pharmacologique antagoniste réceptoriel de TNF-alpha, c'est-à-dire induire une réponse pharmacologique cohérente dans au moins l'un des tests suivants :
   - pour les antagonistes réceptoriels de TNF-alpha : inhibition de l'adhésion de macrophages induite par TNF-alpha sur les cellules endothéliales ou inhibition de la libération d'anions superoxydes induite par TNF-alpha sur les neutrophiles ou inhibition de l'activité mitogène du TNF-alpha sur les fibroblastes du derme.

L'antagoniste de TNF-alpha peut en outre avoir une affinité sélective pour les récepteurs spécifiques de ces composés : TNF-alpha.

Pour qu'une substance soit reconnue comme un antagoniste de la libération et/ou de la synthèse de TNF-alpha, elle doit répondre notamment à la caractéristique suivante :
- inhibition de la libération d'interleukine 1 ou de TNF-alpha par des monocytes (cellules U937) différenciés par un ester de phorbol (PMA).

Les antagonistes de TNF-alpha sont actuellement testés pour traiter la fièvre, le choc septique, la cachexie.

Les antagonistes de l'invention sont notamment des composés comprenant au moins un hétérocycle et des composés azotés comprenant au moins un cycle benzénique.

Les antagonistes réceptoriels de TNF-alpha et les inhibiteurs de la libération et/ou de la synthèse de TNF-alpha utilisables dans l'invention sont en particulier la lisophyline, l'A802715, la sulfasalazine.

Les antagonistes de TNF-alpha peuvent être synthétisés ou extraits de produits naturels (végétaux ou animaux).

Dans les compositions selon l'invention, les antagonistes de TNF-alpha sont utilisés de préférence en une quantité allant de 0,000001 à 5 % en poids par rapport au poids total de la composition, et en particulier en une quantité allant de 0,0001 à 0,1 % en poids par rapport au poids total de la composition.

Les compositions selon l'invention peuvent se présenter sous toutes les formes galéniques normalement utilisées pour une application topique, notamment sous forme de solutions aqueuses, hydroalcooliques ou huileuses, de dispersions du type lotion ou sérum, de gels anhydres ou lipophiles, d'émulsions de consistance liquide ou semi-liquide du type lait, obtenues par dispersion d'une phase grasse dans une phase aqueuse (H/E) ou inversement (E/H), ou de suspensions ou d'émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

Elles peuvent être également utilisées pour le cuir chevelu sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires ou mieux après solaires, des lotions, gels ou mousses pour le soin de la peau, comme des lotions de nettoyage ou de désinfection, des lotions anti-solaires, des lotions de bronzage artificiel, des compositions pour le bain, des compositions désodorisantes contenant un agent bactéricide, des gels ou lotions après-rasage, des crèmes épilatoires, des compositions contre les piqûres d'insectes, des compositions anti-douleur ou des compositions pour traiter certaines maladies de la peau comme celles citées précédemment.

Les compositions selon l'invention peuvent également consister en des préparations solides constituant des savons ou des pains de nettoyage.

Les compositions peuvent aussi être conditionnées sous forme de composition pour aérosol contenant également un agent propulseur sous pression.

Les antagonistes de TNF-alpha peuvent être aussi incorporés dans diverses compositions pour soins ou traitements capillaires, et notamment des shampooings éventuellement antiparasitaires, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, etc.

Les compositions de l'invention peuvent aussi être à usage bucco-dentaire, par exemple une pâte dentifrice ou un bain de bouche. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans les domaines cosmétique, pharmaceutique ou dermatologique. L'émulsionnant et le coémulsionnant sont présents, dans la composition, en une proportion allant de 0,3 % à 30 % en poids, et de préférence de 0,5 à 30 % ou mieux de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % du poids total de la composition.

De façon connue, la composition de l'invention peut contenir également des adjuvants habituels dans le domaine cosmétique, pharmaceutique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine cosmétique, pharmaceutique ou dermatologique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, ou encore l'éthylcellulose, le polyéthylène.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon et des extraits végétaux, notamment ceux d'Aloe Vera.

Comme actifs lipophiles, on peut utiliser le rétinol (vitamine A) et ses dérivés, le tocophérol (vitamine E) et ses dérivés, les acides gras essentiels, les céramides, les huiles essentielles.

On peut entre autres associer les antagonistes de TNF-alpha à des agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir ;
- les agents kératolytiques tels que les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide tartrique, l'acide citrique et de manière générale les acides de fruits et les bêta-hydroxyacides comme l'acide salicylique et ses dérivés notamment alcoylés comme, l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

De façon avantageuse, les antagonistes de TNF-alpha sont associés à des produits à effet secondaire irritant et notamment des actifs, utilisés couramment dans le domaine cosmétique, pharmaceutique ou dermatologique. La présence d'un antagoniste de TNF-alpha dans une composition cosmétique, pharmaceutique ou dermatologique contenant un produit voire un actif ayant un effet irritant permet d'atténuer fortement, voire de supprimer cet effet irritant.

En particulier, l'antagoniste de TNF-alpha permettent notamment d'augmenter la quantité d'actif cosmétique, pharmaceutique ou dermatologique par rapport à la quantité normalement utilisée, en vue d'une efficacité améliorée.

Aussi, l'invention a encore pour objet une composition contenant dans un milieu cosmétiquement, pharmaceutiquement ou dermatologiquement acceptable au moins un produit à effet secondaire irritant, caractérisée en ce qu'elle contient au moins un agent antagoniste de cet effet choisi parmi les antagonistes de TNF-alpha et leurs associations.

Elle a aussi pour objet l'utilisation dans une composition topique contenant un milieu cosmétiquement, pharmaceutiquement ou dermatologiquement acceptable et au moins un produit à effet secondaire irritant, d'au moins un composé choisi parmi les antagonistes de TNF-alpha et leurs associations, pour éliminer cet effet irritant.

Les produits irritants auxquels s'applique l'invention sont notamment les parfums, les tensioactifs (ioniques ou non-ioniques), les conservateurs, certains filtres solaires, les solvants organiques, les solutions alcooliques et certains actifs cosmétiques, pharmaceutiques ou dermatologiques.

En particulier, les actifs à effet secondaire irritant sont choisis parmi les α-hydroxy-acides (glycolique, lactique, malique, citrique, tartrique, mandélique ), les β-hydroxy-acides (acide salicylique et ses dérivés), les α-céto-acides, les β-céto-acides, les rétinoïdes (rétinol et ses esters, rétinal, acide rétinoïque et ses dérivés, rétinoïdes, notamment ceux décrits dans les documents FR-A-2 570 377, EP-A-199636, EP-A-325540, EP-A-402072), les anthralines (dioxyanthranol), les anthranoïdes, les peroxydes (notamment de benzoyle), le minoxidil, les sels de lithium, les antimétabolites, la vitamine D et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les dépigmentants (hydroquinone), les actifs antipoux (pyréthrine).

L'emploi d'antagoniste de TNF-alpha permet notamment de multiplier de 2 à 10 fois la quantité de produit, et plus spécialement d'actif à effet secondaire irritant par rapport à l'état de la technique, sans ressentir tous les inconforts mentionnés ci-dessus. Ainsi, on peut utiliser les hydroxyacides jusqu'à 50 % du poids de la composition ou les rétinoïdes jusqu'à 5 %, sans aucune gène.

En particulier, la composition contient un ou plusieurs antagonistes de TNF-alpha choisi parmi les alcoxy- et/ou aryloxy-tétrazolyl-benzofuranne-carboxamides ou un alcoxy- et/ou aryloxy-tétrazol-yl-benzothiophène-carboxamides et un ou plusieurs actifs à effet secondaire irritant choisis parmi les alpha-hydroxyacides et les bêta-hydroxyacides.

Le procédé de traitement cosmétique peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions après-solaires sur la peau ou sur les cheveux secs, application d'une lotion pour cheveux sur cheveux mouillés, de shampooings ou encore application de dentifrice sur les gencives.

Les exemples suivants illustrent l'invention. Dans ces exemples, les proportions indiquées sont des pourcentages en poids.

### Exemple 1 : lotion démaquillante pour le visage qui ne fait pas partie de l'invention

| | |
|---|---|
| Loratidine | 0,05 |
| Antioxydant | 0,05 |
| Isopropanol | 40,00 |
| Conservateur | 0,30 |
| Eau | qsp 100 % |

### Exemple 2: lotion démaquillante pour le visage qui ne fait pas partie de l'invention

| | |
|---|---|
| Cétirizine | 0,001 |
| Antioxydant | 0,05 |
| Isopropanol | 40,00 |
| Conservateur | 0,30 |
| Eau | qsp 100 % |

### Exemple 3 : Gel pour le soin du visage, qui ne fait pas partie de l'invention

| | |
|---|---|
| Auranofine | 0,05 |
| Hydroxypropylcellulose (Klucel H vendu par la société Hercules) | 1,00 |
| Antioxydant | 0,05 |
| Isopropanol | 40,00 |
| Conservateur | 0,30 |
| Eau | qsp 100 % |

### Exemple 4 : Gel pour le soin du visage

| | |
|---|---|
| Lisophyline | 0,04 |
| Hydroxypropylcellulose (Klucel H vendu par la société Hercules) | 1,00 |
| Antioxydant | 0,05 |
| Isopropanol | 40,00 |
| Conservateur | 0,30 |
| Eau | qsp 100 % |

### Exemple 5 : Crème de soin du visage (émulsion huile dans eau)

| | |
|---|---|
| Sulfasalazine | 0,02 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

### Exemple 6 : Shampooing traitant, qui ne fait pas partie de l'invention

| | |
|---|---|
| Loratadine | 0,02 |
| Hydroxypropylcellulose (Klucel H vendu par la société Hercules) | 1,00 |
| Parfum | 0,50 |
| Conservateur | 0,30 |
| Eau | qsp 100 % |

### Exemple 7 : Crème de soin antirides pour le visage (émulsion huile dans eau) qui ne fait pas partie de l'invention

| | |
|---|---|
| Cétirizine | 0,15 |
| Stéarate de glycérol | 2,00 |
| Polysorbate 60 (Tween 60 vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,50 |
| Conservateur | 0,30 |
| Eau | qsp 100 % |

### Exemple 8 : Gel émulsionné de soin contre les piqûres d'insectes (émulsion huile dans eau), qui ne fait pas partie de l'invention

| | |
|---|---|
| Cyclomethicone | 3,00 |
| Huile de Purcellin (vendue par la Société Dragocco) | 7,00 |
| PEG-6/PEG-32/Glycol Stéarate (Tefose^{R} 63 de Gattefosse) | 0,30 |
| Sétastine | 0,02 |
| Conservateur | 0,30 |
| Parfum | 0,40 |
| Carbomer | 0,60 |
| Crotamiton | 5,00 |
| Acide glycyrrhétinique | 2,00 |
| Alcool éthylique | 5,00 |
| Triéthanolamine | 0,20 |
| Eau | qsp 100 % |

### Exemple 9 : Gel anti-douleur qui ne fait pas partie de l'invention

| | |
|---|---|
| Cétirizine | 0,03 |
| Hydroxypropylcellulose (Klucel H vendu par la société Hercules) | 1,00 |
| Antioxydant | 0,05 |
| Chlorhydrate de lidocaïne | 2,00 |
| Isopropanol | 40,00 |
| Conservateur | 0,30 |
| Eau | qsp 100 % |

### Exemple 10 : Crème de soin de l'érythème solaire (émulsion huile dans eau) qui ne fait pas partie de l'invention

| | |
|---|---|
| Dexchlorphéniramine | 0,25 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau | qsp 100 % |

### Exempte 11 : Crème de soin antirides pour le visage (émulsion huile-dans-eau) qui ne fait pas partie de l'invention

Cet exemple se différencie de l'exemple 7, par le remplacement de la cétirizine par le 3-benzyloxy-5-méthoxy-N-1H-tétrazol-5-yl-benzothiophène-2-carboxamide fabriqué selon l'exemple 1 du document EP-A-299457 et l'acide n-octanoyl-5-salicylique par l'acide glycolique.

### Exemple 12 : Crème de soin antirides pour le visage (émulsion huile dans eau) qui ne fait pas partie de l'invention

Cet exemple se différencie de l'exemple 7, par le remplacement de la sulfasalazine par le 5-méthoxy-3-phénoxy-N-1H-tétrazol-5-yl-benzothiophène-2-carboxamide fabriqué selon l'exemple 2 du document EP-A-2995457 et l'acide n-octanoyl-5-salicylique par un mélange d'acide de fruit (acides lactique, glycolique, tartrique, citrique, malique).

### Exemple 13 : Gel pour traiter l'acné, qui ne fait pas partie de l'invention

| | |
|---|---|
| Acide all trans rétinoïque | 0,05 |
| Loratidine | 0,55 |
| Hydroxypropylcellulose (Klucel H vendu par la société Hercules) | 1 |
| Antioxydant | 0,05 |
| Isopropanol | 40 |
| Conservateur | 0,3 |
| Eau | qsp 100 % |

## Revendications

1. Composition topique contenant dans un milieu cosmétiquement, pharmaceutiquement ou dermatologiquement acceptable au moins un produit à effet secondaire irritant, **caractérisée en ce qu'**elle contient au moins un agent antagoniste de cet effet choisi parmi les antagonistes de TNF-alpha et leurs associations.

2. Composition selon la revendication précédente, **caractérisée en ce que** le produit à effet secondaire irritant est choisi parmi les α-hydroxy-acides, les β-hydroxy-acides, les α-céto-acides, les β-céto-acides, les rétinoïdes, les anthralines, les anthranoïdes, les peroxydes, le minoxidil, les sels de lithium, les antimétabolites, la vitamine D et ses dérivés, les dépigmentants, les solvants, les parfums, les conservateurs, les tensioactifs, les solutions alcooliques.

3. Composition selon la revendication 1 ou 2, **caractérisée en ce qu'**elle contient en outre au moins un agent choisi parmi les agents antibactériens, antiparasitaires, antiviraux, antifongiques, anti-inflammatoires, antiprurigineux, anesthésiques, kératolytiques, antiradicaux libres, anti-séborrhéiques, antipelliculaires, anti-acnéiques et/ou les agents modulant la différenciation et/ou la prolifération et/ou la pigmentation cutanée.

4. Composition selon la revendication 3, **caractérisée en ce que** les α-hydroxy-acides sont utilisés jusqu'à 50% du poids de la composition ou les rétinoïdes jusqu'à 5% du poids de la composition.

5. Composition suivant la revendication 1, **caractérisée en ce que** les antagonistes de TNF-alpha sont choisis parmi les antagonistes réceptoriels de TNF-alpha, les inhibiteurs de la libération et/ou de la synthèse de TNF-alpha.

6. Compsition suivant l'une quelconques des revendications 1 et 5, caratérisée en ce que l'antagoniste de TNF-alpha est choisi parmi la lisophyline, l'A802715, la sulfasalazine.

## Patentansprüche

1. Zusammensetzung zur topischen Anwendung, die in einem kosmetisch, pharmazeutisch oder dermatologisch akzeptablen Medium mindestens ein Produkt mit reizender Nebenwirkung enthält, **dadurch gekennzeichnet, dass** sie mindestens einen Antagonisten gegen diese Wirkung enthält, der unter den Antagonisten von TNF-α und ihren Kombinationen ausgewählt ist.

2. Zusammensetzung nach dem vorhergehenden Anspruch, **dadurch gekennzeichnet, dass** das Produkt mit reizender Wirkung unter den α-Hydroxysäuren, β-Hydroxysäuren, α-Ketosäuren, β-Ketosäuren, Retinoiden, Anthralinen, Anthranoiden, Peroxiden, Minoxidil, Lithiumsalzen, Antimetaboliten, Vitamin D und seinen Derivaten, depigmentierenden Wirkstoffen, Lösungsmitteln, Parfums, Konservierungsmitteln, grenzflächenaktiven Stoffen und alkoholischen Lösungen ausgewählt ist.

3. Zusammensetzung nach Anspruch 1 oder 2, **dadurch gekennzeichnet, dass** sie ferner mindestens einen Wirkstoff enthält, der unter den antibakteriellen Wirkstoffen, antiparasitären Wirkstoffen, antiviralen Wirkstoffen, Antimykotika, entzündungshemmenden Wirkstoffen, juckreizstillenden Wirkstoffen, anästhesierenden Wirkstoffen, Keratolytika, Radikalfängern für freie Radikale, Antiseborrhoeika, Antischuppenmitteln, Wirkstoffen gegen Akne und/oder Mitteln, die die Differenzierung und/oder Proliferation und/oder Pigmentierung der Haut verändern, ausgewählt ist.

4. Zusammensetzung nach Anspruch 3, **dadurch gekennzeichnet, dass** die α-Hydroxysäuren in einer Menge von bis zu 50 % des Gewichts der Zusammensetzung oder die Retinoide in einer Menge von bis zu 5 % des Gewichts der Zusammensetzung verwendet werden.

5. Zusammensetzung nach Anspruch 1, **dadurch gekennzeichnet, dass** die Antagonisten von TNF-α unter den Rezeptorantagonisten von TNF-α, Inhibitoren der Freisetzung von TNF-α und/ oder Inhibitoren der Synthese von TNF-α ausgewählt sind.

6. Zusammensetzung nach einem der Ansprüche 1 und 5, **dadurch gekennzeichnet, dass** der Antagonist von TNF-α unter Lisophyllin, A802715 und Sulfasalazin ausgewählt ist.

## Claims

1. Topical composition containing, in a cosmetically, pharmaceutically or dermatologically acceptable medium, at least one product with an irritant side effect, **characterized in that** it contains at least one antagonist of this effect, chosen from TNF-α antagonists and combinations thereof.

2. Composition according to the preceding claim, **characterized in that** the product with an irritant side effect is chosen from α-hydroxy acids, β-hydroxy acids, α-keto acids, β-keto acids, retinoids, anthralins, anthranoids, peroxides, Minoxidil, lithium salts, antimetabolites, vitamin D and its derivatives, depigmenting agents, solvents, fragrances, preserving agents, surfactants and alcoholic solutions.

3. Composition according to Claim 1 or 2, **characterized in that** it also contains at least one agent chosen from antibacterial agents, antiparasitic agents, antiviral agents, antifungal agents, antiinflammatory agents, anti-pruriginous agents, anaesthetics, keratolytic agents, free-radical scavengers, anti-seborrhoeic agents, antidandruff agents, antiacne agents and/or agents for modifying cutaneous differentiation and/or proliferation and/or pigmentation.

4. Composition according to Claim 3, **characterized in that** the α-hydroxy acids are used at up to 50% of the weight of the composition, or the retinoids at up to 5% of the weight of the composition.

5. Composition according to Claim 1, **characterized in that** the TNF-α antagonists are chosen from TNF-α receptor antagonists and inhibitors of TNF-α release and/or synthesis.

6. Composition according to either of Claims 1 and 5, **characterized in that** the TNF-α antagonist is chosen from lisophyline, A802715 and sulfasalazine.
